# EUROPEAN PATENT SPECIFICATION

(11) **EP 0 349 138 B1**
(45) Date of publication and mention of the grant of the patent: **24.08.1994**
(21) Application number: 89305826.3
(22) Date of filing: 09.06.1989
(51) Int. Cl.: C07C 403/24, C07C 403/02, C09B 61/00

(54) **Recovery of carotenoids**
Rückgewinnung von Carotenoiden
Obtention de caroténoides

(30) Priority: 15.06.1988 AU 8770/88
(43) Date of publication of application: 03.01.1990
(73) Proprietor: PALM OIL RESEARCH & DEVELOPMENT BOARD, 43000 Kajang Selangor (MY)
(72) Inventor: Cheng Keat, Ooi, 13050 Butterworth (MY); Yuen May, Choo, 58100 Kuala Lumpur (MY); Augustine Soon Hock, Ong, Kuala Lumpur (MY)
(74) Representative: Votier, Sidney David

(56) References cited:
- BE-A- 556 855
- GB-A- 1 515 238
- GB-A- 2 160 874

## Description

This invention relates to the process of producing carotenoid concentrates and has particular but not exclusive application to the process of producing carotene concentrates from palm oil.

### Background Art

Carotenoids are the principal oil soluble yellow to orange red pigments found in plants and animals. Generally, there are two classes of carotenoids, that is the carotenes and xanthophylls. Carotenes are hydrocarbon carotenoids while xanthophylls are oxygenated carotenoids. Carotenoids are highly unsaturated compounds and are easily decomposed by heat, light and oxygen.

The more widely known carotenes are the alpha-, beta- and gamma-carotenes and lycopene. Some of the carotenes are precursors of vitamin A. Beta-carotene is a precusor of vitamin A and has also been shown to inhibit tumour progression, hence reduces cancer formation. As the carotenes are natural compounds and with their vitamin A property, it is widely used in commercial applications in the pharmaceutical and nutritional products .

The most important sources of carotenes are from vegetables, fruits and vegetable oil such as red palm oil. Among these sources, palm oil is the richest sources of carotenes. The orange red colour of palm oil is due to the presence of the carotenes. The concentration of carotenes in palm oil can range from 500 ppm to 3000 ppm, depending on the species of the palm fruit from which the oil is obtained. The commercial red palm oil contains about 500-700 ppm of carotenes of which alpha-and beta- carotene formed up to 90% of the total carotenes. So far commercial - production of natural carotenes comes mainly from carrots, while commercial extraction of carotenes from palm oil is not very successful.

Some of the common techniques used in the recovery of carotenes from palm oil are saponification followed by extraction, adsorbent method, extraction by selective solvents, crystallisation and molecular distillation of carotenes from the oil. In most of these techniques, large scale production of the carotenes from palm oil will be very expensive and involved too many steps in the process.

A method of recovering carotene concentrate is for example disclosed in GB-A-1515238.

### Summary of the Invention

The present invention accordingly provides a simple process for the recovery of carotenes and the production of carotene concentrates from palm oil which comprises the steps of mixing esterified palm oil containing carotenes, or other solution containing carotenes, with an edible oil, part by part of the mixture and subjecting the resulting mixture to a pressure of less than 8.0 x 10⁻⁵ bar (0.060 Torr) and a temperature of less than 200°C, without incurring substantial decomposition of the carotenes during the process.

The temperature is preferably in the range 50 to 200°C, more preferably 70 to 190°C. The pressure is preferably in the range 2.7 x 10⁻⁶ to 8.0 x 10⁻⁵ bar (0.002 to 0.060 Torr). The percentage of edible oil added to the esterified oil containing carotenes or the other solution containing carotenes is preferably in the range 0.1% to 50%, more preferably 1% to 10%, parts by weight of the mixture.

According to one preferred embodiment of the invention, the esterified oil containing carotenes or the other solution containing carotenes is mixed with 0.1% to 50% parts by weight of an edible oil and passing the resulting mixture through a distillation apparatus, preferably at a temperature range of 50 to 200°C and a pressure range 2.7 x 10⁻⁶ to 8.0 x 10⁻⁵ bar (0.002 to 0.060 Torr).

The esterified oil containing carotenes or the other solution containing carotenes can be the alkyl esters such as the methyl esters, ethyl esters, isopropyl esters or butyl esters of the fatty acids of palm oil and its products, or any other food grade solvents that made up the solution. The edible oil used is preferably palm oil and its products, such as palm olein, palm stearin, neutralized palm oil, neutralized palm olein, or other vegetable oils. In this process, the alkyl esters of the fatty acids of palm oil or its products, or the other food grade solvents of the solution containing the carotenes, in the mixture are distilled off, while the carotenes are concentrated in the edible oil fraction. The carotenes concentrate is dark red in colour. The carotenes in the concentrates or edible oil is substantially stable during over a long period of time and have a concentration of at least 1000 ppm and above. The concentration of the carotenes can range from 1000 ppm to 30000 ppm or higher depending on the concentration of the starting material or proportion of edible oil that is added to the esterified oil containing carotenes or solution containing carotenes.

Preferably, the mixture is heated to the required temperature and then allowed to pass through a distillation apparatus where the temperature of the mixture is maintained and the required pressure created. The distilled alkyl esters or food grade solvents are collected in a separate container from the carotene concentrate.

Preferably the esterified oil or esterified palm oil containing the carotenes are prepared via esterification or transesterification of palm oil or oils containing carotenes. The oil used in the esterification or transesterification is preferably palm oil, palm olein, or palm stearin or any other vegetable oil containing carotenes. The alkyl esters produced by the esterification or transesterification process are preferably the methyl, ethyl, isopropyl or butyl esters of the fatty acids. The edible oil added to the esterified oil containing carotenes or solution containing carotenes is preferably palm oil and palm oil products such as palm olein and palm stearin, other vegetable oils such as peanut, soyabean, corn, rapeseed, sunflower, olive, palmkernel, coconut and fish oils, in each case, either crude, degummed and bleached, refined bleached and deodorised or refined.

The present invention will now be illustrated by the following examples.

### Example 1

200 parts by weight of crude palm oil having a concentration of 645 ppm and free fatty acids of less than 5% was transesterified with 79 to 120 parts by weight of methanol and 0.5 to 1.0 parts by weight of a base catalyst. After the transesterification reaction, the ester was separated from the glycerol, and washed with water until the washing was neutral. The ester was then dried using drying agents or vacuum. The dried ester was still orange red in colour and has a carotene content of 700 ppm.

200 parts by weight of dried ester was then added with 20 parts by weight of refined and deodorised (RD) red palm oil. The resulting mixture was then passed through a vacuum distillation column(molecular distillator) at a pressure of 2.7 x 10⁻⁵ to 3.3 x 10⁻⁵ bar (0.002 to 0.025 Torr) and a temperature of 90°C. The concentration of carotene of the concentrate obtained after the distillation was 6570 ppm.

The experiment was repeated using different temperatures for distillation and the results are shown in Table 1.

**TABLE 1**

| | | | | |
|---|---|---|---|---|
| Temperature (°C) | 110 | 130 | 150 | 170 |
| Carotene Concentrate (ppm) | 6650 | 6687 | 6754 | 6985 |

### Example 2

200 parts by weight of the dried ester as prepared in Example 1 was added with 10 parts by weight of refined and deodorised (RD) red palm oil. The resulting mixture was then passed through a vacuum distillation column(molecular distillator) at a pressure of 2.7 x 10⁻⁵ to 3.3 x 10⁻⁵ bar (0.020 to 0.025 Torr) and a temperature of 90°C. The concentration of carotene of the concentrate obtained after the distillation was 9673 ppm.

The experiment was repeated using different temperatures for distillation and the results are shown in Table 2.

**TABLE 2**

| | | | | |
|---|---|---|---|---|
| Temperature (°C) | 110 | 130 | 150 | 170 |
| Carotene Concentrate (ppm) | 9754 | 9799 | 9861 | 9980 |

### Example 3

200 parts by weight of the dried ester as prepared in Example 1 was added with 5 parts by weight of refined and deodorised (RD) red palm oil. The resulting mixture was then passed through a vacuum distillation column(molecular distillator) at a pressure of 2.7 x 10⁻⁵ to 3.3 x 10⁻⁵ bar (0.020 to 0.025 Torr)and a temperature of 90°C. The concentration of carotene of the concentrate obtained after the distillation was 18762 ppm.

The experiment was repeated using different temperatures for distillation and the results are shown in Table 3.

**TABLE 3**

| | | | | |
|---|---|---|---|---|
| Temperature (°C) | 110 | 130 | 150 | 170 |
| Carotene Concentrate (ppm) | 18798 | 18861 | 18890 | 18994 |

### Example 4

200 parts by weight of crude palm oil having a concentration of 645 ppm and free fatty acids of less than 5% was transesterified with 79 to 135 parts by weight of ethanol and 0.5 to 1.0 parts by weight of a base catalyst. After the transesterification reaction, the ester was separated from the glycerol, and washed with water until the washing was neutral. The ester was then dried using drying agents or vacuum. The dried ester was still orange red in colour and has a carotene content of 705 ppm.

200 parts by weight of dried ester was then added with 20 parts by weight of refined and deodorised (RD) red palm oil. The resulting mixture was then passed through a vacuum distillation column(molecular distillator) at a pressure of 2.7 x 10⁻⁵ to 3.3 x 10⁻⁵ bar (0.020 to 0.025 Torr)and a temperature of 90°C. The concentration of carotene of the concentrate obtained after the distillation was 6508 ppm.
The experiment was repeated using different temperatures for distillation and the results are shown in Table 4.

**TABLE 4**

| | | | | |
|---|---|---|---|---|
| Temperature (°C) | 110 | 130 | 150 | 170 |
| Carotene Concentrate (ppm) | 6597 | 6637 | 6761 | 6887 |

### Example 5

200 parts by weigh of the dried ester as prepared in Example 4 was added with 10 parts by weight of refined and deodorised (RD) red palm oil. The resulting mixture was then passed through a vacuum distillation column(molecular distillator) at a pressure 2.7 x 10⁻⁵ to 3.3 x 10⁻⁵ bar (0.020 to 0.025 Torr) and a temperature of 90°C. The concentration of carotene of the concentrate obtained after the distillation was 9558 ppm.

The experiment was repeated using different temperatures for distillation and the results are shown in Table 5.

**TABLE 5**

| | | | | |
|---|---|---|---|---|
| Temperature (°C) | 110 | 130 | 150 | 170 |
| Carotene Concentrate (ppm) | 9617 | 9695 | 9782 | 9901 |

### Example 6

200 parts by weight of the dried ester as prepared in Example 4 was added with 5 parts by weight of refined and deodorised (RD) red palm oil. The resulting mixture was then passed through a vacuum distillation column(molecular distillator) at a pressure of 2.7 x 10⁻⁵ to 3.3 x 10⁻⁵ bar (0.020 to 0.025 Torr)and a temperature of 90°C. The concentration of carotene of the concentrate obtained after the distillation was 18409 ppm.

The experiment was repeated using different temperatures for distillation and the results are shown in Table 6.

**TABLE 6**

| | | | | |
|---|---|---|---|---|
| Temperature (°C) | 110 | 130 | 150 | 170 |
| Carotene Concentrate (ppm) | 18515 | 18590 | 18695 | 18741 |

### Example 7

200 parts by weight of the dried ester as prepared in Example 4 was added with 5 parts by weight of refined and deodorised (RD) red palm oil. The resulting mixture was then passed through a vacuum distillation column(molecular distillator) at a pressure of 2.7 x 10⁻⁶ to 5.3 x 10⁻⁶ bar (0.002 to 0.004 Torr) and a temperature of 150°C. The concentration of carotene of the concentrate obtained after the distillation was 20783 ppm.

### Example 8

200 parts by weight of the dried ester as prepared in Example 4 was added with 5 parts by weight of refined bleached, and deodorised(RBD) palm olein. The resulting mixture was then passed through a vacuum distillation column(molecular distillator) at a pressure of 2.7 x 10⁻⁵ to 3.3 x 10⁻⁵ bar of (0.020 to 0.025 Torr) and a temperature of 130°C. The concentration of carotene of the concentrate obtained after the distillation was 18571 ppm.

### Example 9

200 parts by weight of the dried ester as prepared in Example 4 was added with 5 parts by weight of neutralised, bleached and degummed (NBD) palm oil. The resulting mixture was then passed through a vacuum distillation column (molecular distillator) at a pressure of 2.7 x 10⁻⁵ to 3.3 x 10⁻⁵ bar (0.020 to 0.025 Torr) and a temperature of 90°C. The concentration of carotene of the concentrate obtained after the distillation was 19048 ppm.

### Example 10

200 parts by weight of the dried ester as prepared in Example 4 was added with 5 parts by weight of refined bleached and deodorised (RBD) palm oil. The resulting mixture was then passed through vacuum distillation column (molecular distillator) at a pressure of 2.7 x 10⁻⁵ to 3.3 x 10⁻⁵ bar (0.020 to 0.025 Torr) and a temperature of 130°C. The concentration of carotene of the concentrate obtained after the distillation was 18650 ppm.

### Example 11

200 parts by weight of the dried ester as prepared in Example 4 was padded with 5 parts by weight of refined and deodorised (RD) red palm olein. The resulting mixture was then passed through a vacuum distillation column (molecular distillator) at a pressure of 2.7 x 10⁻⁵ to 3.3 x 10⁻⁵ bar (0.020 to 0.025 Torr) and a temperature of 130°C. The concentration of carotene of the concentrate obtained after the distillation was 19708 ppm.

## Claims

1. A process for the recovery of carotenes substantially without destroying the carotenes present in the carotene concentrate which comprises the steps of transesterification of oil containing carotenes and vacuum distillation characterized by the addition of 0.1% to 50% of edible oil to the transesterified oil containing carotenes or solution containing carotenes and the resulting mixture to a pressure of less than 8.0 x 10⁻⁵ bar (0.060 Torr) and a temperature of less than 200 degree Celsius.

2. A process as claimed in Claim 1, wherein the oil used in the transesterification process is crude palm oil, crude palm olein, crude palm stearin, neutralized palm oil or neutralised palm olein.

3. A process as claimed in Claim 1 or 2, wherein the transesterified oil containing carotenes is the methyl esters, the ethyl esters or the isopropyl esters of the fatty acids.

4. A process as claimed in Claim 1 or 2, wherein the solution containing carotenes is any food grade solvent or mixture of food grade solvents.

5. A process as claimed in any of Claims 1 to 4, wherein the edible oil added to the transesterified oil containing carotenes or solution containing carotenes is crude palm oil, refined and deodorised (RD) red palm oil, refined, bleached and deodorised (RBD) palm oil, neutralised, bleached and degummed (NBD) palm oil, crude palm olein, neutralized, bleached and degummed (NBD) palm olein, refined, bleached and deodorised (RBD) palm olein, refined and deodorised (RD) red palm olein, neutralized palm oil, neutralized palm olein, or refined, bleached and deodorised palm stearin.

6. A process as claimed in any of Claims 1 to 4, wherein the edible oil added to the transesterified oil containing carotenes or solution containing carotenes is soyabean oil, corn oil, rapeseed oil, sunflower oil, olive oil, peanut oil, palmkernel oil, coconut oil, fish oil or carrot oil, or a mixture of any two or more of them.

7. A process as claimed in any of the preceding claims, wherein the amount of edible oil added to the transesterified oil containing carotenes or solution containing carotenes is in the range of 1% to 10% of the resulting mixture.

8. A process as claimed in any of the preceding claims, wherein the process of distillation of the resulting mixture is carried out at a temperature in the range of 70 to 190 degree Celsius.

9. A process as claimed in any of the preceding claims, wherein the distillation of the resulting mixture is carried out at a pressure in the range of 2.7 x 10⁻⁶ to 8.0 x 10⁻⁵ bar (0.002 to 0.060 Torr).

## Patentansprüche

1. Verfahren zur Rückgewinnung Von Carotinen im wesentlichen ohne Zerstörung der in dem Carotinekonzentrat vorhandenen Carotine, wobei das Verfahren die Schritte der Umesterung von Carotine-enthaltendem Öl und Vakuumdestillation umfaßt, gekennzeichnet durch den Zusatz von 0,1 bis 50 % eßbarem Öl zu dem Carotine-enthaltenden umgeesterten Öl oder zu der Carotine-enthaltenden Lösung und Einwirkenlassen eines Drucks von weniger als 8,0 x 10⁻⁵ bar (0,060 Torr) und einer Temperatur von weniger als 200 °C auf das sich ergebende Gemisch.

2. Verfahren nach Anspruch 1, wobei das in dem Umesterungsverfahren verwendete Öl rohes Palmöl, rohes Palmolein, rohes Palmstearin, neutralisiertes Palmöl oder neutralisiertes Palmolein ist.

3. Verfahren nach Anspruch 1 oder 2, wobei das Carotine-enthaltende umgeesterte Öl die Methylester, Ethylester oder lsopropylester der Fettsäuren ist.

4. Verfahren nach Anspruch 1 oder 2, wobei die Carotine-enthaltende Lösung irgendein Nahrungsmittel-geeignetes Lösungsmittel oder ein Gemisch von Nahrungsmittel-geeigneten Lösungsmitteln ist.

5. Verfahren nach einem der Ansprüche 1 bis 4, wobei das eßbare Öl, das zu dem Carotine-enthaltenden umgeesterten Öl oder zu der Carotine-enthaltenden Lösung hinzugefügt worden ist, rohes Palmöl, raffiniertes und desodoriertes (RD) rotes Palmöl, raffiniertes, gebleichtes und desodoriertes (RBD) Palmöl, neutralisiertes, gebleichtes und entschleimtes (degummed) (NBD) Palmöl, rohes Palmolein, neutralisiertes, gebleichtes und entschleimtes (NBD) Palmolein, raffiniertes, gebleichtes und desodoriertes (RBD) Palmolein, raffiniertes und desodoriertes (RD) rotes Palm-olein₁ neutralisiertes Palmöl, neutralisiertes Palmolein oder raffiniertes, gebleichtes und desodoriertes Palmstearin ist.

6. Verfahren nach einem der Ansprüche 1 bis 4, wobei das eßbare Öl, das zu dem Carotine-enthaltenden umgeesterten Öl oder zu der Carotine-enthaltenden Lösung hinzugefügt worden ist, Sojabohnenöl, Mais-öl, Rapsöl, Sonnenblumenöl, Olivenöl, Erdnußöl, Palmkernöl, Kokosnußöl, Fischöl oder Karottenöl oder ein Gemisch von zweien oder mehreren von diesen ist.

7. Verfahren nach einem der vorhergehenden Ansprüche, wobei die Menge des eßbaren Öls, das zu dem Carotine-enthaltenden umgeesterten Öl oder zu der Carotine-enthaltenden Lösung hinzugefügt worden ist, im Bereich von 1 bis 10 % des sich ergebenden Gemischs liegt.

8. Verfahren nach einem der vorhergehenden Ansprüche, wobei das Destillationsverfahren des sich ergebenden Gemischs bei einer Temperatur im Bereich von 70 bis 190 °C durchgeführt wird.

9. Verfahren nach einem der vorhergehenden Ansprüche, wobei die Destillation des sich ergebenden Gemischs bei einem Druck im Bereich von 2,7 x 10⁻⁶ bis 8,0 x 10⁻⁵ bar (0,002 bis 0,060 Torr) durchgeführt wird.

## Revendications

1. Procédé de récupération des carotènes, essentiellement sans détruire les carotènes présents dans le concentré de carotène, qui comprend les étapes de transestérification d'une huile contenant des carotènes et à procéder à une distillation sous vide, caractérisé par l'addition de 0,1 à 50 % d'une huile comestible à l'huile transestérifiée contenant des carotènes ou à une solution contenant des carotènes, le mélange obtenu étant soumis à une pression inférieure à 8,0.10⁻⁵ bar (0,060 Torr) et à une température inférieure à 200°C.

2. Procédé selon la revendication 1, dans lequel l'huile utilisée dans l'opération de transestérification est l'huile de palme brute, 1'oléine de palme brute, la stéarine de palme brute, l'huile de palme neutralisée ou l'oléine de palme neutralisée.

3. Procédé selon la revendication 1 ou 2, dans lequel l'huile transestérifiée contenant des carotènes est représentée par les esters méthyliques, les esters éthyliques ou les esters isopropyliques d'acides gras.

4. Procédé selon la revendication 1 ou 2, dans lequel la solution contenant des carotènes est représentée par un solvant quelconque de qualité alimentaire ou un mélange de solvants de qualité alimentaire.

5. Procédé selon l'une quelconque des revendications 1 à 4, dans lequel l'huile comestible ajoutée à l'huile transestérifiée contenant des carotènes ou une solution contenant des carotènes est l'huile de palme brute, l'huile de palme rouge raffinée et désodorisée (RD), l'huile de palme raffinée, blanchie et désodorisée (RBD), l'huile de palme neutralisée, blanchie et dégommée (NBD), l'oléine de palme brute, l'oléine de palme neutralisée, blanchie et dégommée (NBD), l'oléine de palme raffinée, blanchie et désodorisée (RBD), l'oléine de palme rouge raffinée et désodorisée (RD), l'huile de palme neutralisée, l'oléine de palme neutralisée, ou encore la stéarine de palme raffinée, blanchie et désodorisée.

6. Procédé selon l'une quelconque des revendications 1 à 4, dans lequel l'huile comestible ajoutée à l'huile transestérifiée contenant des carotènes ou une solution contenant des carotènes est l'huile de soja, l'huile de maïs, l'huile de colza, l'huile de tournesol, l'huile d'olive, l'huile d'arachide, l'huile de palmiste, l'huile de coprah, l'huile de poisson ou l'huile de carotte, ou un mélange d'au moins deux d'entre elles.

7. Procédé selon l'une quelconque des revendications précédentes, dans lequel la quantité d'huile comestible ajoutée à l'huile transestérifiée contenant des carotènes ou une solution contenant des carotènes est comprise entre 1 et 10 % par rapport au mélange final.

8. Procédé selon l'une quelconque des revendications précédentes, dans lequel l'opération de distillation du mélange final est mise en oeuvre à une température comprise entre 70 et 190°C.

9. Procédé selon l'une quelconque des revendications précédentes, dans lequel la distillation du mélange obtenu est réalisée sous une pression de 2,7.10⁻⁶ à 8,0.10⁻⁵ bar (de 0,002 à 0,060 Torr).
